# EUROPEAN PATENT SPECIFICATION

(11) **EP 1 244 910 B1**
(45) Date of publication and mention of the grant of the patent: **02.03.2005**
(21) Application number: 00985630.3
(22) Date of filing: 20.12.2000
(51) Int. Cl.: G01N 33/487, C12M 1/34

(54) **APPARATUS FOR AND METHOD OF MAKING ELECTRICAL MEASUREMENTS ON OBJECTS**
VORRICHTUNG UND VERFAHREN ZUR AUSFÜHRUNG ELEKTRISCHER MESSUNGEN AN GEGENSTÄNDEN
APPAREIL ET PROCEDE DESTINES A REALISER DES MESURES ELECTRIQUES SUR DES OBJETS

(30) Priority: 24.12.1999 GB 9930718
(43) Date of publication of application: 02.10.2002
(73) Proprietor: AstraZeneca AB, S-151 85 Södertälje (SE)
(72) Inventor: DODGSON, John, Croydon Surrey CR2 7JP (GB); THOMSEN, Lars, 9210 Aalborg (DK)
(74) Representative: Bill, Kevin
(86) International application number: PCT/GB2000/004887
(87) International publication number: WO 2001/048474

(56) References cited:
- WO-A-94/25862
- WO-A-97/17426
- WO-A-99/31503
- DE-A- 19 712 309
- US-A- 4 055 799

## Description

This invention relates to an apparatus for, and method of, making electrical measurements on cells, liposomes or similar small objects, in a medium. More particularly the invention relates to an apparatus for, and method of, making electrophysiological measurements on cells, liposomes or similar small objects, in a medium.

Previously electrical measurements on small objects such as cells, liposomes or similar small objects, such as membrane fragments, have been made using an electrolyte-filled micropipette or similar apparatus, brought into contact with the object. A seal is made between the object and the tip of the micropipette such that electrolyte inside the pipette contacts the object. A high electrical resistance is thereby achieved in the vicinity of the seal, and the measurement made by monitoring current and electrical potential between a first electrode, contacting the electrolyte, and a second electrode, contacting a liquid bathing the object. The conventional procedure involves delicate manipulation and is labour-intensive and prone to failure. The present invention is intended to overcome this and other problems associated with presently existing apparatus and method. WO 99/31503 discloses devices and methods of the "patch-clamp" type in which substantially planar micro-machined structures having an aperture against which a cell is sealed are used.

According to the present invention there is provided an apparatus and a method for making electrical measurements on an object in a medium, the object being a cell, liposome or similar small object, as defined in the appended independent claims.

Preferably a characteristic dimension of a channel through, or along, which an object passes or flows is of the order of 50 µm, more preferably it is less than 25 µm. Preferably a characteristic dimension of the orifice to which the object seals is of the order of 10 microns, more preferably less than 5 microns.

Objects or cells are introduced into a chamber entrained in liquid by way of a pump or gravity feed or other suitable liquid displacement mechanism, for example by electro-osmosis.

The feature to which the object is sealed comprises an orifice through which a liquid contacts the object. Preferably the orifice has a shape, and is formed from a material which allow the object to seal readily to a sealing surface around the orifice. In particular, the orifice is preferably hollowed or tapered to allow the object to deform into the orifice so as to fit to the taper. Preferably the area to which the object is intended to be sealed is coated with a material which will improve the seal. Most preferably that material is a thin film of glass, for example borosilicate glass, deposited in the region of the orifice.

Electrodes are provided to make contact with liquid in a channel leading to the orifice, and to the liquid surrounding the object on the other side of the orifice. In a preferred embodiment at least one of these electrodes is integrated into the structure. For convenience the combination of orifice and electrodes, when configured to measure impedance, are hereinafter referred to as test positions.

Means for measuring the electrical impedance between the electrodes are adapted to detect the presence of a cell or other object in the vicinity of the orifice, and to monitor the presence and quality of a seal between the object and the area surrounding the orifice, as well as the electrical properties of the object when exposed to controlled amounts of chemical species in the solutions on either side of the orifice.

Preferably there is provided a plurality of the aforementioned test positions arranged in an array. An advantage of such an array is that many objects may be acted upon in parallel. This increases throughput. An array of test positions may be formed on a semiconductor substrate, such as for example, silicon. Proximity detectors, electrodes and processing means may be included on the substrate, for example, in a different layer of an integrated semiconductor structure.

Means for locating each object with respect to a test position may comprise a mechanical or electrical structure. An example is a well or well-like structure, which may be formed, for example, by back etching a silicon substrate in which the object locates. A mesh or structure with a sieving action can be placed at the exit of the well so as to permit passage of fluid but prevent the object from leaving the well. Preferably a pressure differential established across the substrate urges objects into the well-like structures at each test position.

As more objects are located the pressure differential increases because less wells are available, through which fluid may flow. Consequently the pressure differential increases. This increased pressure tends to force objects into the wells if they deform relatively easily. A means is preferably provided to obtain an indication of wells which are occupied and to use this information to reduce or increase the pressure differential. The means may be a pressure differential measuring device such as a manometer, or it could be a serial checking device which inspects test positions separately.

Preferably the apparatus is microfabricated from a biocompatible material. The microfabricated apparatus may include one or more microfabricated channels. These may be formed for example by etching silicon. Wells or seal positions may be at a locus in a fluid flow channel.

The use of channels to lead the objects to the test positions is advantageous in that the need for precise manipulation by an operator is removed. Preferably fluid flow arrangements in the apparatus are such that the objects will be carried to the test positions by the fluid flow, and localised there ready for tests without further actuation within the apparatus. Optionally however other forms of actuation might be employed, for example, electrical or mechanical actuation, for example by dielectrophoretic movement, or piezoelectric mechanical actuation.

The channel is preferably narrow, for example, between 1 and 5 times the diameter (which may typically be around 5 - 20 µm) of the objects to be tested. Alternatively the channel or well may be relatively wide except for a constriction in the region at which location the test position lies. In a particularly preferred embodiment fluid pathways may be formed in a location which prevents cells or objects from being forced through an orifice at a test position. The pathway may be formed by back etching or otherwise as hereindescribed.

In a microfabricated device electronic logic may be used to monitor the location of objects at the test positions and to control the process of establishing and maintaining the seal, and then to measure the electrical characteristics of the object. Logic circuitry may be integrated within a semiconducting substrate, for example using CMOS, DMOS or bi-polar components, fabricated in a convenient process sequence. Preferably the substrate also forms a support for microfabricated channels. Post-processing techniques can be used during manufacture of the substrate to interconnect electronic components to electrodes in flow channels or at test positions.

The introduction of electronic components or logic circuitry, by an active substrate technique, is elegant and is of especial benefit when an array or arrays of test positions are co-fabricated on a common substrate. The possibility exists to substitute or augment such components or circuitry by attaching additional microelectronic components, at appropriate positions, to the substrate. Such components may be attached by surface mount, die and wire bonding, TAB bonding or flip chip bonding. The attachment of devices using conductive adhesive means is especially preferred since this minimises any thermal stresses imparted to the structure during fabrication.

Preferred devices and attachment means are attached by surface mount (including attachment by conductive adhesives) or by wire bonding. The aforementioned devices are particularly preferred where the substrate is passive or contains low voltage components. Analogue processing circuitry, analogue-to-digital converters, digital signal processing devices, microcomputing or microcontroller elements, and communications devices may also be integrated onto the substrate. The latter devices include optical communication devices. Integration facilitates connection of processing or control circuitry to external processors, such as a microprocessor for closed loop flow control and/or measurement of electrical parameters.

Optionally the processing means responds to an external indication of the presence or state of an object at the test position. The indication may in turn be derived by image processing means such as a video microscope image of the channel, to detect the presence of a cell to be tested.

Integrated components or circuitry and an associated well or channel, or each test position, are provided with a unique address and a communication means is provided allowing communication to and from a microprocessor. Preferably communication is via a common link or bus.

If the support substrate is silicon the possibility exists to view the cell handling structures through the silicon using suitable infra red radiation. In such an embodiment care must be taken in the layout of the structures to prevent obscuration of the radiation path. An advantage of this embodiment is that the device need not use any member which is optically transmissive in the normal visible band, but is infra red transparent.

Means may be provided to remove objects that have been tested from the test positions. Such removal may be achieved in the case of a cell, liposome or membrane fragment by introduction of an agent to dissolve the membrane material, which will then be removed from the sealing surface allowing a subsequent seal to be made to the sealing surface.

In cases where the test apparatus has electrical connections routed about or around it, for example in a highly integrated active substrate, it may be desirable to provide electrical guard bands suitably disposed around portions of the fluid handling structure so that any electric field applied to the fluid is reduced sufficiently so that there is minimal interference and cross-talk between measurements in different parts of the structure.

Optical components, such as waveguide optics, may be integrated in processed layers of the substrate which are preferably fabricated in a similar manner to those defining fluid channels. Such optical components may include waveguides for interrogation of the cell or support medium in the fluid channel. These may include evanescent field coupling. Alternatively optical components communicate to external signal processing means. Optical components include structures interfacing with fibre optic elements such as etched silicon V-grooves.

Arrays of apparatuses may utilise common external connections for supply of fluids, cells and power supplies and may be imaged in parallel using suitable video microscopy means.

Embodiments of the invention will now be described, by way of examples only, and with reference to the following Figures in which:
Figure 1 is a cross section of a first embodiment of an apparatus showing in detail a test position;
Figure 2 is a cross section of a second embodiment of an apparatus showing in detail a test position;
Figure 3 is a cross section of a third embodiment of an apparatus showing in detail a test position;
Figure 4 is a cross section of a fourth embodiment of an apparatus showing in detail a test position;
Figure 5 shows two diagrammatical, sectional views of orifices shaped for improved sealing at the test position
Figure 6 is a cross section of a fifth embodiment of an apparatus showing in detail a test position;
Figure 7 shows in section an embodiment of the invention in which electrical contact is made to the test positions by means of an electrode manipulated by robotics; and
Figure 8 shows a section through a further embodiment of the invention based on the use of a pre-formed mesh structure.

Referring to the figures, for the sake of convenience only the various embodiments and their operation will be described with regard to tests on cells, it being understood that this shall refer also in each case to tests on other similar small objects.

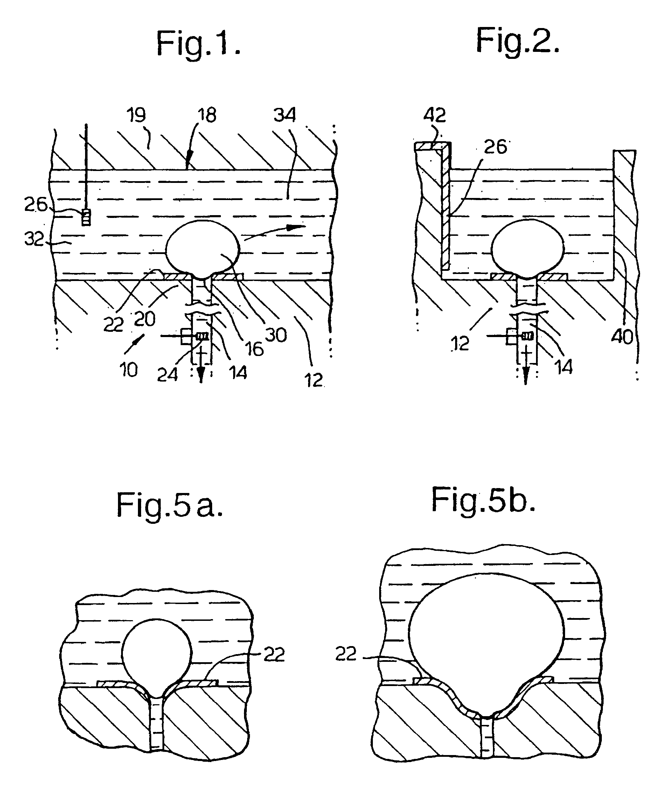

Figure 1 shows a first embodiment of a test position 10 according to the invention, in which a substrate 12 has a channel 14 within it, opening to an orifice 16 at a location at which an object such as a cell is to be tested. The orifice communicates with a channel 18 along which cells are flowed to reach the test location. Around the orifice is a sealing surface 20 which is optionally coated with a material layer 22 to control the sealing effect, usually to enhance it in order to achieve a very high resistance seal between the channel 14 and the channel 18. An example of such a material is a thin film of borosilicate glass. In the event that the substrate 12, or at least the upper layer of 12 nearest the orifice, is of a material to which a good seal can be made, layer 22 can be omitted. Alternatively, layer 22 might be a material that when coated with a further one or more materials introduced through channel 14 or channel 18, then acts to produce the required seal. Layer 22 might be of a material that gives specific adhesion of one or more cell types to a given position, or acts to do this in combination with one or more other chemicals flowed down channel 14 or channel 18. Electrodes 24 and 26 are provided in electrical contact with the liquids in channels 14 and 18 respectively, and are connected to electrical measuring apparatus (not shown) so as to measure the current and voltage between channels 14 and 18, this including the conductivity of any cell 30 present at the test position. In operation cells approach the test position 10 along an inlet channel 32 being moved by flow of liquid in which they are entrained between inlet 32 and outlet 34, or by other means such as dielectrophoresis or centrifugal force. Movement of the liquid in the case that the cells are entrained might be by for example bulk flow in response to pressure differential, or by electroosmotic flow. The cell is then localised at the test position by fluid flow from channel 18 to channel 14, or by other means for example dielectrophoretic drive in a non-uniform AC field imposed between an electrode in channel 14 and a second in channel 18, or between one or more electrodes localised in the vicinity of orifice 16 and in one located in channel 18. The conductivity between channel 14 and channel 18 is monitored to determine the presence of the cell near the orifice 16 and control the localisation force by a feedback mechanism. When localised at the orifice, additional localisation force is applied so that the cell membrane seals to the sealing area 20. The quality of seal is monitored by the impedance between channel 14 and channel 18 and the localisation force is maintained and/or increased, by a feedback control means, until a high impedance (of the order of 10s of megohms or more) is reached, indicating a good seal has been formed. Measurements can then be made, for example of the electrophysiological response of a cell to changing concentrations of chemicals flowing through channel 18 past the cell. This test mode is referred to in electrophysiology as the 'cell attached' mode. Alternatively, the membrane portion located at the orifice 16 might be made permeable to ions or other species in the liquid in channel 14, or removed entirely, by for example an electroporation pulse applied between electrodes in channels 14 and 18, by introduction of a chemical agent to the membrane via channel 14, by a negative pressure pulse in channel 14, or any other means. This will allow measurements to be made in 'whole cell' mode. Further variations of techniques in electrophysiology and patch clamping experiments can be achieved by variations of pressure pulses as will be recognised by those skilled in the art.

When the experiment is over, the cell can be removed e.g. by a sudden positive pressure pulse in channel 14 or increase in flow along channel 18, or by introduction of a cell lysing compound along channels 14 or 18. Residual membrane material attached to the cell sealing area 20 might be removed by treatment with protease or other solvents for membranes, but it is known in the art that it is difficult to achieve a second good seal on a previously used sealing area even when this is cleaned. For this reason the sealing material 22 in area 20 might be renewed for example by dissolving or otherwise modifying the surface layer, or by adding further material which in conjunction with the sealing material 22 acts to form the seal. Alternatively the device comprising the test position 10 might be disposable and intended for a single use only. While only a single position is shown in fig. 1 it is understood that a plurality of such positions are likely to be provided in a device. In this case, means are provided to make electrical measurements separately at each position. A common electrode might be provided in channel 14 and each of the electrodes 26 in channels 18 be electrically isolated from the others by means of separation of the solutions in the channels (for example by means of slugs of air, or by using entirely separate channels). Alternatively a common electrode 26 might be provided in a common channel 18 leading to each of the positions, and separate channels 14 provided each with a separate electrode.

Figure 2 shows an alternative embodiment similar to that in figure 1 and where the corresponding components have the same nomenclature and function. Here the test position comprises a well 40 into which access of the cells or other objects is from above, each well having a separate electrode 42, e.g. an Ag/AgCl electrode, contacting the liquid in the well. The electrodes 42 are connected to electronics off the chip by means of conventional conductor tracks on the chip and wire bonding to a header as is known in conventional electronics technology. Cells are supplied in liquid into the well, sucked down onto the seal area and electrical measurements made as described above. Liquid and cells might be supplied by dispensing cells in separate aliquots of liquid into each well, or by dispensing a greater number of cells in liquid into a common manifold (not shown) above the wells, followed by suction of liquid through the channel 14, so as to lower the liquid level below the edge of the wells, so isolating the solutions and contacts in the wells. Provision is made in the electrical measurement means to detect isolation between the various electrodes 42, so determining the right degree of suction of liquid.

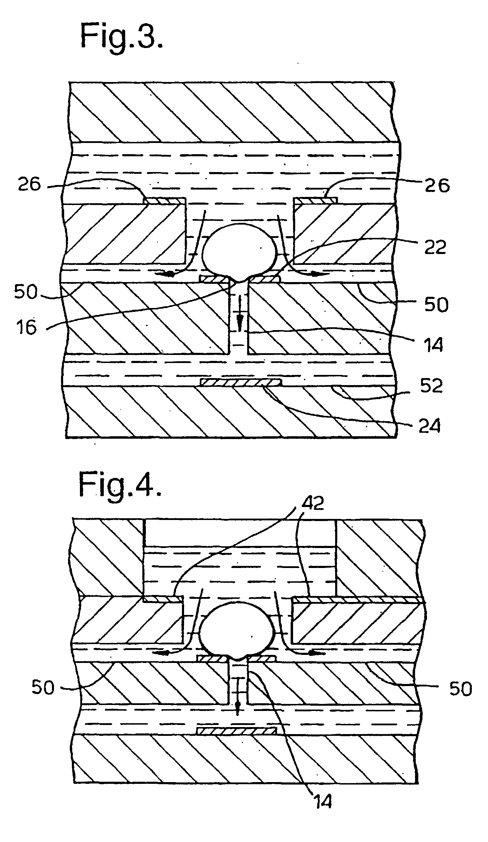

Figure 3 shows a further embodiment of a test position where the test position is in the form of a well, where cells are immobilised in the well as described above, but one or more additional channels 50 are provided leading from the well in order to facilitate suction of the cell into the well from a region above it, that region either being an inlet channel as in the embodiment of fig. 1, or a common cell and liquid supply manifold as in the embodiment of fig. 2. Additional channels 50 are particularly advantageous if the orifice 16 is small so limiting the flow through it. Channels 50 are also advantageous with reversed flow in order to remove the cell. A channel 52 is provided leading from the channel 14, channel 52 acting as a manifold for suction to act on the cell, and to make common electrical contact to the lower side of one or more cells, and to supply chemical agent to the cells as required. The other parts in fig. 3 are as described in figs. 1 and 2, except that here the electrode 24 is shown explicitly in a location in the suction channel 52 - this might be an electrode common to a number of cells in an array-type device - and electrode 26 is shown surrounding the top of each well. This is an advantageous arrangement for impedance measurements and for use of a higher voltage pulse between the electrodes in order to electroporate the cell membrane in the region of orifice 16, as described for fig. 1. Also, this arrangement provides a highly non-uniform field in the region of the orifice, allowing AC dielectrophoretic manipulation of the cell using a field applied to the same electrodes.

Figure 4 shows a further embodiment as described in figure 3, but combined with the arrangement for supply of cells and liquid followed by isolation of liquid in the wells described for the embodiment in fig 2. As described for figure 2, a control means detects the isolation of the electrodes 42 one from another, hence setting the correct level of liquid in the wells. This embodiment avoids a problem which might be encountered in that of fig. 2 in that suction of the liquid can be via channels 50 independently of sealing of the cell to channel 14. In fig. 2, when the cell is sealed, no further liquid can be withdrawn.

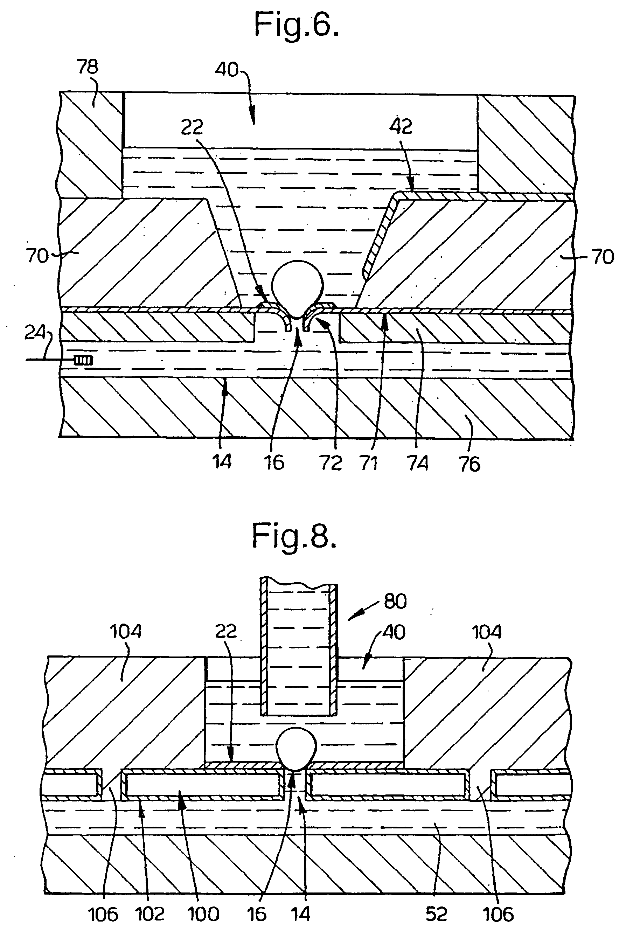

Figure 5 shows a variety of profiles that might be applied to the seal area surrounding the orifice in the above embodiments. The most advantageous will depend on the properties of the cells that are being tested. Fig. 5a shows a profile for a cell that deforms and forms seals easily. Fig. 5b shows a profile for a cell that does these less readily. In each case a seal material 22 can be applied to the seal area and chemically treated as appropriate and as described above for the embodiment of fig. 1.

Figure 6 shows a specific manner of construction of the embodiment shown in fig. 2 in which one or more test positions are formed on a silicon chip. A silicon substrate 70 has formed on it an orifice structure 72 of inverted nozzle configuration which defines an orifice 16. The nozzle structure is formed preferably of a membrane 71 of a dielectric, e.g. silicon nitride, overlying the substrate 70. It is fabricated in a manner described in our copending application [WO0020554]. On the opposite side of the orifice structure from the silicon is formed a series of layers 74 and 76 which together define a channel 14 in communication with orifice 16. An electrode 24 (not shown) is in contact with the liquid in channel 14 as described for the previous embodiments. It is envisaged that if the chip has a plurality of test positions, then the channel 14 will be common to all of them, and the electrode 24 will also be common. It might be mounted off the chip or integrated onto the chip either in channel 14 or in contact with it. The silicon substrate is etched to form a well communicating with the other side of the orifice structure, an area around the orifice then being coated if required with a seal material 22, by for example sputtering a thin film of borosilicate glass. A electrode 42 is formed by sputtering, electroplating or otherwise depositing a layer of silver metal either alone, or as a coating on an underlying conductor, followed by a process of chloridisation to form an Ag/AgCl electrode. Electrode 42 is electrically connected to a conductor track (not shown) and thereby to a contact to make electrical connection off the chip in a conventional manner. One or more additional plastic layers 78 are provided to further define the well 40. The apparatus is used as described for figures 1 and 2.

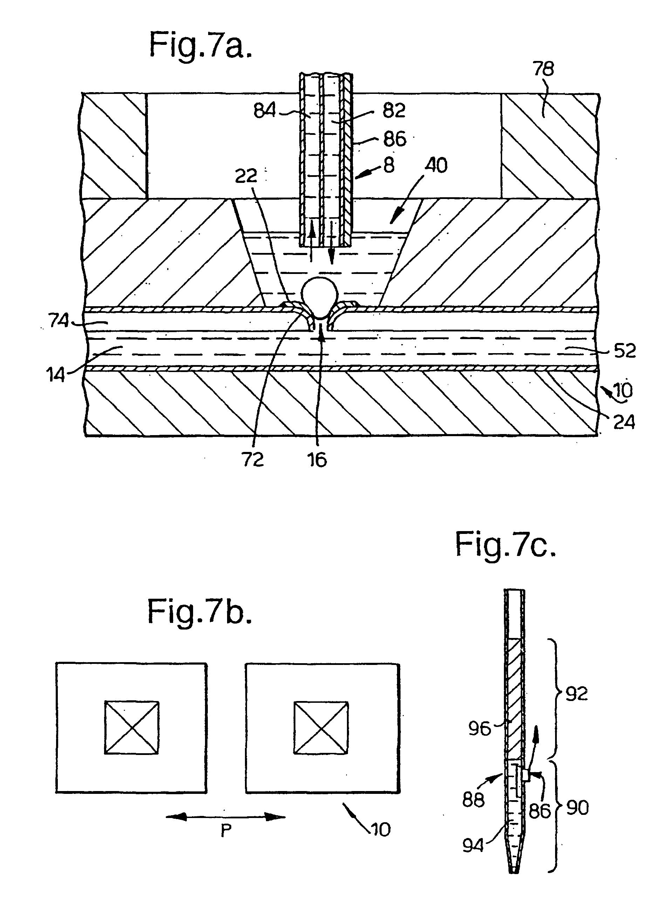

Figure 7 shows a further embodiment of the invention which is a variation on the embodiment shown in fig. 6, showing delivery of liquid and cells to the well and their removal by means of one or more capillaries mounted on a robotic head. In fig. 7 a combined solution supply and contact means 80 is mounted on a robot head and aligned with each of a number of wells in succession to deliver cells, deliver liquids and make contact to the liquid in the well. Means 80 comprises a solution supply capillary 82 and an electrode 86, preferably an Ag/AgCl electrode, and an optional solution withdrawal capillary 84. Electrode 86 acts to replace the electrode 42 in the embodiment shown in fig. 2. The advantage of the embodiment in fig. 7 is that contact can be made easily to a plurality of test position wells 40 on a chip by an array of contact and liquid supply means 80, without having to engineer contacts as part of the chip. This could result in a substantial cost saving and hence promote use of the chip as a disposable single use device. Preferably the electrode 86 might contact the liquid supply capillary 82 instead of making contact directly to the liquid in the well 40. This would allow a shorter length of electrode wire to be used and simplify the construction of means 80, possibly decreasing susceptibility of the apparatus to noise. Optionally liquid might be supplied to the well and then withdrawn through a single capillary 82, though preferably a second capillary 84 or a common drain channel similar to that shown as reference 50 in fig. 4 is provided to give fast removal of liquid from around the cell under test. Test compounds may then be delivered sequentially to the cell through the capillary by means of a multi way valve and sampler system (not shown). Alternatively compounds might be delivered to the cell by aspiration into capillary 82 from a microtitre plate as in conventional robotic liquid handling. In this case the wells are advantageously patterned to correspond to the spacing in a conventional microtitre plate, for example on 96, 384 or 1536 well format, with plastic well surrounds 78 designed to correspond to the design in the respective plates as shown in fig. 7b for two neighbouring test positions 10. It is envisaged that a number of capillary and contact means 80 are provided acting in parallel, as in conventional microtitre plate robotics, except that here the capillary means remain in contact with the solution in the wells after dispensing in order to make electrical contact.

The liquid delivery and contact means might be a capillary and contact arrangement as shown in fig. 7c. If this is done then a correct sequence of aspirations and aspiration volumes can be chosen to reduce the likelihood of cross contamination while making contact to the liquid in the well, and to give continuous electrical measurement while the compound is supplied to the cell. The liquid delivery and contact capillary 88 has an electrode 86 mounted to make contact to liquid inside the capillary, with an electrical contact leading to a measurement device. The volume of the capillary and the position of the electrode are such that when the volume occupying the well 40 is held in the capillary, the electrolyte reaches the contact 86 as shown in fig. 7c, but the capillary can hold twice this volume. Two aliquots 94 and 96 of either the same or different solutions are shown in fig. 7c occupying regions 90 and 92 of the capillary respectively. The filling device can fill either a single (region 90) or double well volume (region 90 plus region 92) into the capillary. Operation of the embodiment in fig. 7a and 7c is as follows. The channel 14 is filled with electrolyte, allowing this to fill into the orifice 16 by capillary action. A double volume of electrolyte containing cells is then aspirated and a single volume deposited into the well. Contact between solution in the well and in channel 14 is checked for and suction applied to channel 14 until a cell is trapped in position. The impedance between electrodes 24 and 86 is monitored to check for a good seal. In a multi-position chip a plurality of capillaries 88 and measurements of cell sealing are used simultaneously. Those that form good seals are used for tests, others are ignored. The capillary 88 is then drained to waste and filled to single volume with a test compound, followed by a single volume of electrolyte. Contact is made to the cell by delivering first the electrolyte to the well, then the compound. The electrolyte establishes a baseline for the cell parameters and continuous measurement is achieved while the compound is delivered to the cell. Solution might be removed by aspiration followed by washout of the well before delivery of the next compound; alternatively solution is removed via a drain channel at the base of the well as described above. The precise volumes deposited are arranged so that the cell is maintained in a small volume of residual solution in the base of the well. Variations on the above procedure might be used to give differing sequences of exposure to solutions.

Figure 8 shows a further embodiment. A mesh 100 is created by techniques known in the art, for example, electroplating around a photopattemed array of resist areas, or by sputtering material between resist areas followed by a lift-off process. Alternative meshes are as supplied for example by Agar Scientific Ltd, of Stansted, UK, where the material 100 is typically of carbon supported on a larger scale metal support structure. The orifices in the mesh are preferably circular, and the spacing of the centres of the orifices preferably greater than two orifice diameters. If the material of which the mesh is fabricated is conductive, then a coating 102 is applied so as to make the surface insulating. This can be done for example, in the case where the mesh is of metal, by oxidation, or in the general case by sputtering or otherwise coating the surface with an insulating adherent coating as known in conventional electronic fabrication. A plastic layer 104 for example of photopatternable epoxy type 'SU8', is then patterned over the mesh using conventional techniques to form a number of wells 40. A step where removable resist is patterned in the area of the wells might be used if there are problems of removing the pattemable plastic from the channel 14. The mesh might be designed so that the orifices are spaced to conform with the spacing of the wells. Alternatively, meshes might be used where the spacing of the orifices is less than the spacing of the wells, in which case the orifices neighbouring the wells are filled by the patterned plastic 104 and so blocked. Indeed, the mesh might be such that the spacing is random. It is necessary that only one orifice is present in each well. However it is envisaged in the invention that more than one might be present in a proportion of the wells, and that these would be recognised in the process of establishing a seal to a cell in each of the wells and those wells ignored. It is envisaged that sufficient redundancy is built into the system that a suitable number of wells with one orifice, and a cell sealed successfully to it, would be achieved. The wells are preferably coated with a seal layer 22 and liquid, cells and compounds to be tested are delivered to the well by a contact and liquid delivery means 80 as described previously. Seals to cells are established by liquid contact to the cell membrane followed by suction in the manifold 52.

The mesh 100 is advantageously of an inorganic material, so that coefficients of expansion of the various coatings are matched and the seal promoting layer 22 adheres readily to the underlying structure. However, mesh 100 might be of organic material provided that a good sealing surface for cells can be established. Provided that temperature excursions can be avoided, this might still be done as in the case of an inorganic mesh, by sputtering a thin film of borosilicate glass onto the surface of the plastic mesh; adhesion between the inorganic coating and the plastic might be enhanced by for example a plasma treatment to the plastic before coating, as is known in the art of manufacture of plastic drinks containers. In this case, plastic filter membranes with randomly arranged pores, for example 'Nucleopore' filter membrane, might be used.

It is understood that while a single test position device has been described in some of the above embodiments, the invention is intended to cover either single or multiple devices, possibly in an array on a common substrate, with separate or common fluidic and electrical connections as may be necessary or advantageous.

## Claims

1. An apparatus for making electrical measurements on an object in a medium, the object being a cell, liposome or similar small object, the apparatus comprising:
a surface (20) with an orifice (16) formed therein, the orifice (16) being adapted so that the object, in use, is capable of substantially sealing the orifice (16) thereby defining first and second cavity portions (14, 18), which cavity portions (14,18) are electrically insulated from each other;
a first electrode (26) which in use is conductively linked with the object in said first cavity portion (18)
a second electrode (24) which in use is conductively linked with the object at a location in said second cavity portion (14), and
means for measuring a variation in the impedance between said electrodes (24,26) in dependence on a variation of a parameter within said second cavity (14);
**characterised in that** the apparatus further comprises:
localisation means for applying a localisation force to the object in order to locate the object at the orifice (16);
means for monitoring the conductivity between the first and second cavity portions (14, 18); and
a feedback mechanism for controlling the localisation force in response to the conductivity monitored by the means for monitoring the conductivity between the first and second cavity portions (14, 18).

2. Apparatus as claimed in claim 1 further comprising a sealant (22) attached to the orifice (16).

3. Apparatus as claimed in claim 1 or claim 2 in which the localisation means comprises a fluid flow arrangement for locating the object at the orifice (16) by fluid flow.

4. Apparatus as claimed in any of claims 1 to 3 further comprising:
means for applying an additional localisation force so that the object seals to a sealing area; and
feedback control means for maintaining and/or increasing the additional localisation force until a high impedance between the first and second cavity portion (14,18) is reached.

5. An apparatus as claimed in any of claims 1 to 4 wherein the orifice (16) tapers from the first cavity (18) to the second cavity (14).

6. A method of making electrical measurements on an object, the object being a cell, liposome or similar small object, the method comprising the steps of:
providing a test position having an orifice (16), and first and second channels (14, 18) communicating with said orifice;
suspending the object in a medium;
introducing the medium to the first channel (18)
locating the object at the test position;
establishing a seal between the object and said orifice (16) so as to define two spaced locations; and
varying the characteristic of the medium at one location so as to cause a variation in the impedance of the object and measuring the change in impedance with respect to a datum;
**characterised in that** the object is located at the test position by applying a localisation force to the object and controlling the localisation force by a feedback mechanism in response to the conductivity measured between the first and second channels (14,18).

## Patentansprüche

1. Vorrichtung zur Durchführung elektrischer Messungen an einem Objekt in einem Medium, wobei das Objekt eine Zelle, ein Liposom oder ein ähnliches kleines Objekt ist und die Vorrichtung folgendes umfasst:
eine Fläche (20) mit einer darin ausgebildeten Öffnung (16), wobei die Öffnung (16) dafür ausgelegt ist, dass das verwendete Objekt in der Lage ist, die Öffnung (16) im Wesentlichen abzudichten, wodurch erste und zweite Hohlraumteile (14, 18) gebildet werden, die elektrisch voneinander isoliert sind,
eine erste Elektrode (26), die im Gebrauch mit dem Objekt im ersten Hohlraumteil (18) leitend verbunden ist,
eine zweite Elektrode (24), die im Gebrauch mit dem Objekt an einem Ort im zweiten Hohlraumteil (14) leitend verbunden ist, und
Mittel zum Messen der Variation der Impedanz zwischen den Elektroden (24, 26) in Abhängigkeit von der Variation eines Parameters in dem zweiten Hohlraum (14),
**dadurch gekennzeichnet, dass** die Vorrichtung außerdem folgendes umfasst:
Lokalisierungsmittel zum Anlegen einer lokalen Kraft an das Objekt, um das Objekt an der Öffnung (16) zu lokalisieren,
Mittel zum Überwachen der Leitfähigkeit zwischen dem ersten und zweiten Hohlraumteil (14, 18) und
einen Rückkopplungsmechanismus zur Regelung der Lokalisierungskraft als Reaktion auf die durch di Mitteln zum Überwachen der Leitfähigkeit überwachte Leitfähigkeit zwischen dem ersten und zweiten Hohlraumteil (14, 18).

2. Vorrichtung nach Anspruch 1, außerdem mit einem an der Öffnung (16) angebrachten Dichtungsmittel (22).

3. Vorrichtung nach Anspruch 1 oder Anspruch 2, wobei das Lokalisierungsmittel eine Fluidströmungsanordnung zum Lokalisieren des Objekts an der Öffnung (16) durch einen Fluidströmung umfasst.

4. Vorrichtung nach einem der Ansprüche 1 bis 3, außerdem mit:
Mitteln zum Anlegen einer zusätzlichen Lokalisierungskraft, so dass das Objekt einen Dichtungsbereich abdichtet, und
Regelungsmittel zur Aufrechterhaltung und/oder Erhöhung der zusätzlichen Lokalisierungskraft bis eine hohe Impedanz zwischen dem ersten und zweiten Hohlraumteil (14, 18) erreicht ist.

5. Vorrichtung nach einem der Ansprüche 1 bis 4, wobei die Öffnung (16) sich von dem ersten Hohlraum (18) zu dem zweiten Hohlraum (14) verjüngt.

6. Verfahren zur Durchführung elektrischer Messungen an einem Objekt in einem Medium, wobei das Objekt eine Zelle, ein Liposom oder ein ähnliches kleines Objekt ist und das Verfahren folgende Schritte umfasst:
Bereitstellen einer Testposition mit einer Öffnung (16) und einem ersten und zweiten Kanal (14, 18), die mit der Öffnung in Verbindung stehen,
Suspendieren des Objekts in einem Medium,
Einführen des Mediums in den ersten Kanal (18),
Lokalisieren des Objekts in der Testposition,
Herstellung einer Abdichtung zwischen dem Objekt und der Öffnung (16), um zwei beabstandete Orte zu definieren, und
Variieren der Eigenschaften des Mediums an einem Ort, um eine Variation der Impedanz des Objekts zu bewirken, und Messen der Impedanzänderung relativ zu einer gegebenen Größe,
**dadurch gekennzeichnet, dass** das Objekt in der Testposition durch das Anlegen einer Lokalisierungskraft an das Objekt und Regelung der Lokalisierungskraft durch einen Rückkopplungsmechanismus als Reaktion auf die Leitfähigkeit, die zwischen dem ersten und zweiten Kanal (14, 18) gemessen wird.

## Revendications

1. Appareil destiné à réaliser des mesures électriques sur un objet dans un milieu, l'objet étant une cellule, un liposome ou un petit objet similaire, l'appareil comprenant :
une surface (20) dans laquelle est pratiqué un orifice (16), l'orifice (16) étant adapté de telle manière que, durant l'utilisation, l'objet est capable de sceller substantiellement l'orifice (16), définissant de la sorte une première et une deuxième parties de cavités (14, 18), lesquelles parties de cavités (14, 18) sont isolées électriquement l'une de l'autre ;
une première électrode (26) qui durant l'utilisation est liée de manière conductrice avec l'objet dans ladite première partie de cavité (18) ;
une deuxième électrode (24) qui durant l'utilisation est liée de manière conductrice avec l'objet à un emplacement dans ladite deuxième partie de cavité (14) ; et
un moyen pour mesurer une variation dans l'impédance entre lesdites électrodes (24, 26) en fonction d'une variation d'un paramètre dans ladite deuxième cavité (14) ;
**caractérisé en ce que** l'appareil comprend en outre :
un moyen de localisation pour appliquer une force de localisation sur l'objet afin de localiser l'objet à l'orifice (16) ;
un moyen destiné à surveiller la conductivité entre les première et deuxième parties de cavités (14, 18) ; et
un mécanisme de feedback pour contrôler la force de localisation en réponse à la conductivité surveillée à l'aide du moyen destiné à surveiller la conductivité entre les première et deuxième parties de cavités (14, 18).

2. Appareil selon la revendication 1 comprenant en outre un moyen d'étanchéité (22) fixé à l'orifice (16).

3. Appareil selon la revendication 1 ou la revendication 2, dans lequel le moyen de localisation comprend un agencement d'écoulement fluide pour localiser l'objet à l'orifice (16) au moyen de l'écoulement fluide.

4. Appareil selon l'une quelconque des revendications 1 à 3 comprenant en outre :
un moyen pour appliquer une force supplémentaire de localisation de telle façon que l'objet est scellé dans une zone étanche ; et
un moyen de contrôle du feedback pour maintenir ou accroître la force supplémentaire de localisation jusqu'à ce qu'une impédance suffisante soit obtenue entre la première et la deuxième parties de cavités (14, 18).

5. Appareil selon l'une quelconque des revendications 1 à 4, dans lequel l'orifice (16) se rétrécit de la première cavité (12) à la deuxième cavité (14).

6. Procédé destiné à réaliser des mesures électriques sur un objet, l'objet étant une cellule, un liposome ou un petit objet similaire, le procédé comprenant les étapes de :
fourniture d'une position d'essai ayant un orifice (16), et un premier et une deuxième canaux (14, 18) communicant avec ledit orifice ;
suspension de l'objet dans un milieu ;
introduction du milieu dans le premier canal (18) ;
localisation de l'objet à la position d'essai ;
établissement d'une étanchéité entre l'objet et ledit orifice (16) de manière à définir deux emplacements espacés ; et
variation des caractéristiques du milieu à un emplacement de manière à causer une variation de l'impédance de l'objet et mesure du changement d'impédance par rapport à des données de référence ;
**caractérisé en ce que** l'objet est situé à la position d'essai en appliquant une force de localisation à l'objet et que la force de localisation est contrôlée par un mécanisme de feedback en réponse à la conductivité mesurée entre les premier et deuxième canaux (14, 18).
